# EUROPEAN PATENT APPLICATION

(11) **EP 4 431 520 A1**
(43) Date of publication of application: **18.09.2024**
(21) Application number: 23173421.1
(22) Date of filing: 15.05.2023
(51) Int. Cl.: C07K 1/113, C07K 14/765

(54) **METHOD FOR THE PREPARATION OF THIOL-ENRICHED ALBUMIN AND USES RELATED THERETO AND THEREOF**

(30) Priority: 16.03.2023 EP 23162385
(71) Applicant: Octapharma AG, 8853 Lachen (CH)
(72) Inventor: Schön, Friedrich, 1100 Vienna (AT); Eder, Markus, 1100 Vienna (AT)
(74) Representative: Ullrich & Naumann PartG mbB

(57) **Abstract**

The present invention relates to human albumin solutions and in particular to methods and uses of preparing them. The invention further relates to human albumin solutions, which have an increased mercaptalbumin content and/or an increased transport capacity for reactive oxygen species, reactive nitrogen species as well as endogenous and exogenous ligands. Embodiments of the invention have been particularly developed for use in the treatment of disease and will be described hereinafter with reference to this application. However, it will be appreciated that the invention is not limited to this particular field of use.

## Description

### FIELD OF THE INVENTION

The present invention relates to human albumin solutions and in particular to methods and uses of preparing them. The invention further relates to human albumin solutions, which have an increased mercaptalbumin content and/or an increased transport capacity for reactive oxygen species, reactive nitrogen species as well as endogenous and exogenous ligands. Embodiments of the invention have been particularly developed for use in the treatment of disease and will be described hereinafter with reference to this application. However, it will be appreciated that the invention is not limited to this particular field of use.

### BACKGROUND OF THE INVENTION

Any discussion of the background art throughout the specification should in no way be considered as an admission that such art is widely known or forms part of common general knowledge in the field.

Human albumin is the most abundant protein in plasma and is the major carrier of endogenous and exogenous ligands, in particular fatty acids (FAs), as well as nucleic acids, hormones, metals, toxins and drugs. It is responsible for most of the pro- and antioxidant capacity of plasma and has (pseudo) enzymatic properties. Fatty acids are the physiological ligands of human albumin, which is characterised by nine fatty acid binding sites, FA1-FA9. Fatty acids bind to the FA1 to FA9 sites with varying degrees of affinity: FA2, FA4 and FA5 are high affinity sites, FA1 and FA3 have medium affinity and FA6 and FA7 have low affinity. HSA plays an important role as a drug carrier (e.g. for antibiotics, anticoagulants, antineoplastics, antivirals, anaesthetics, anxiolytics and non-steroidal anti-inflammatory drugs) by influencing their pharmacokinetics and pharmacodynamics. The FA1, FA3-FA4 and FA7 pockets are the major drug binding sites of HSA. FA1 recognises antibiotics, antineoplastics, antiretrovirals and non-steroidal anti-inflammatory drugs. Ibuprofen is the prototypical ligand of the FA3-FA4 pocket, which also recognises anaesthetics, anxiolytics and the orphan drug 4-phenylbutazone. The FA7 site is considered the primary binding site of HSA. The prototypical ligand is warfarin, a bulky heterocyclic anticoagulant. Several drugs co-bind to the FA7 site in the presence of myristate. This suggests that myristate induces one or more conformational changes in the binding site, resulting in the formation of three non-overlapping secondary sites capable of recognising anaesthetics, anticoagulants, antineoplastics, antiretrovirals and non-steroidal anti-inflammatory drugs (NSAIDs).

Further, human albumin is a valuable biomarker for many diseases (e.g. cancer, rheumatoid arthritis, ischaemia, obesity and diabetes) and has clinical applications in the treatment of several pathologies including shock, trauma, haemorrhage, acute respiratory distress syndrome, haemodialysis, acute liver failure, chronic liver disease and hypoalbuminemia.

In recent years, there has been significant interest in the redox state of thiols in the context of disease. In human plasma, the largest thiol pool is again provided via human albumin. Human albumin comprises 585 amino acids in total (giving the molecule a weight of about 66 KD) and contains 35 Cys residues. However, it only contains a single cysteine (Cys-34), which is not involved in intramolecular disulfide bonds. As the primary plasma protein, albumin has been studied as a major target for oxidative modification and its oxidised form or its redox state have been discussed frequently. While there are several ways to oxidize albumin Cys-34 is a particularly redox-sensitive site within albumin and, as such, one can refer to the redox state of albumin in terms of the redox state of Cys-34. The Cys-34 redox state defines three fractions that generally allow for the determination of the overall albumin redox state. Namely, mercaptalbumin, containing a free thiol group, nonmercaptalbumin-1, containing a disulfide, and nonmercaptalbumin-2, containing a sulfinic or sulfonic acid group.

In plasma, mercaptalbumin conveys several benefits, including:
- antioxidant protection: mercaptalbumin can act as an antioxidant, helping to protect cells and tissues from damage caused by reactive oxygen species (such as H₂O₂, O₂⁻, HOCl, etc.) and reactive nitrogen species (such as ONOO-, ONOOCO₂⁻, etc.) ;
- detoxification: mercaptalbumin can bind to and help remove toxic substances from the body, such as heavy metals and drugs; and
- immune function: mercaptalbumin can help to stimulate the immune system and protect against infections.

Consequently, a high percentage of non-mercaptalbumin can lead to a decreased ability to protect against oxidative damage, detoxify harmful substances, and/or support immune function. Additionally, a low level of mercaptalbumin may be indicative of certain health conditions or diseases, such as liver disease or malnutrition.

For example, nonmercaptalbumin-1 is a reversible derivative whereas nonmercaptalbumin-2 is irreversible. In healthy young people, about 70 to 80% of all albumin is mercaptalbumin, with nonmercaptalbumin-1 making up about 20 to 30%, and nonmercaptalbumin-2 makes up only about 2 to 5%. Increased nonmercaptalbumin-1 levels in plasma are associated with a decreased human albumin anti-oxidative activity. Nonmercaptalbumin-1 and Nonmercaptalbumin-2 are related to the progression of inflammatory processes whereas the concentration of mercaptalbumin in blood plasma is inversely correlated to atherosclerotic damages in the carotid artery of Japanese residents.

WO 2013/024100 A2 describes methods for diagnosing and treating medical conditions associated with oxidative stress, in particular liver diseases. The methods are based on determination and quantification of the fraction of albumin that is irreversibly oxidized at Cys-34. The therapeutic concept suggested incorporates contacting of patient's plasma with an adsorbent capable of binding nonmercaptalbumin-2 followed by infusion of fresh albumin.

Using HPLC Sogami et al., 1985 ("High-performance liquid chromatographic studies on non-mercapt to mercapt conversion of human serum albumin"; Journal of Chromatography, 332, 19-27), determined the relative occurrence of mercaptalbumin and non-mercaptalbumin in plasma of patients suffering from various liver diseases. The signals of a patient's plasma were compared to the signals of a plasma incubated with glutathione at 25°C of the same patient. The more prominent signal of the incubated plasma was thus identified as prolonging to mercaptalbumin.

A similar analytical approach is known from Oettl and Marsche, 2010 ("Redox state of human serum albumin in terms of Cysteine-34 in health and disease"; Methods in Enzymology, Volume 474, 181-195), determining different oxidation steps of albumin, namely mercaptalbumin, non-mercaptalbumin 1 and non-mercaptalbumin 2, in plasma of patients suffering from diabetes, liver damage or renal damage.

Various approaches to modify albumin, whether isolated from plasma or recombinantly-produced, namely by producing conjugates of albumin utilizing the thiol reactivity of Cys-34 have been described. An exemplary process for producing conjugates of albumin is known from WO 2007/071068 A1. The process comprises producing and purifying recombinant albumin and covalently binding a compound to the Cys-34 thiol of albumin. An intermediate enrichment of Cys-34 thiol is suggested by contacting albumin with thioglycolic acid (TGA) or dithiothreitol (DTT).

US 2013/329127 A1 describes modification of thiol groups of various proteins, including human serum albumin, with PEG-Maleimide. A diluted albumin solution is incubated with Tris[2-carboxyethyl]phosphine hydrochloride (TCEP) for 10 minutes prior to addition of a branched PEG reagent containing a terminal maleimide group. After reaction in the dark the obtained PEG-albumin conjugate is further processed and purified.

WO 2007/049941 A1 is concerned with bioactive albumin conjugates, wherein the bioactive compound is covalently bound to albumin via a disulphide bridge. In the course of further analysis, the disulphide bridge is broken up again by reaction of the albumin conjugate with DTT at 37°C.

With respect to disease, WO 2020/104458 A1 describes methods and kits for detecting liver dysfunction in a subject. In WO 2020/104458 A1 the relative abundance of mercaptalbumin, non-mercaptalbumin-1 and non-mercaptalbumin-2 in a healthy person is disclosed as being in the range of 70-80% mercaptalbumin, 20-30% non-mercaptalbumin-1 and <5% non-mercaptalbumin-2, which are ranges also disclosed in Oettl and Marsche, 2010, cited above.

As human albumin is an extremely robust protein with respect to pH stability (stable in the pH range of 4-9), temperature stability (can be heated at 60 °C for up to 10 h in the presence of stabilizers), and even towards organic solvent exposure, numerous human albumin products exist. Typical clinical uses of human albumin products, whether purified from pooled donor plasma on an industrial scale or recombinantly produced are treatment of severe hypoalbuminemia or as a plasma expander.

However, commercial human albumin products have been described as comprising lower amounts of mercaptalbumin and higher amounts of both non-mercaptalbumin-1 and non-mercaptalbumin-2 compared to the respective amounts found in fresh plasma. Further, commercially available albumins are formulated with substantial amounts (i.e. an excess) of fatty compounds such as tryptophan and/or caprylic acid. As a result, the ratio between albumin:fatty compounds in commercially available albumin solutions is routinely between 1:5 and 1:8. For example, the commercially available 5% Octalbin albumin solution comprises 5% albumin (i.e. 50 g/l; 0.75 mmol) as well as tryptophan and caprylic acid each at concentrations of up to 4.2 mmol. The 25% Octalbin albumin solution comprises 25% albumin (i.e. 250 g/l; 3.8 mmol) as well as tryptophan and caprylic acid each at concentrations of up to 21 mmol.

Accordingly, there is a need in the art for improved commercial human albumin products and methods for their production.

It is an object of the present invention to overcome or ameliorate at least one of the disadvantages of the prior art, or to provide a useful alternative. In particular, it is an object of the present invention to provide improved methods for the preparation of a human albumin solution from pooled donor plasma.

### SUMMARY OF THE INVENTION

The above-stated problem is solved by the invention disclosed herein. Specifically, the disclosed methods for the preparation of a human albumin solution from pooled donor plasma, having an increased mercaptalbumin content and/or an increased transport capacity for reactive oxygen species, reactive nitrogen species as well as endogenous and exogenous ligands as well as the related uses and albumin solutions prepared solve the above problem.

Accordingly, in a first aspect, the present invention relates to a method for the preparation of a human albumin solution having an increased mercaptalbumin content and/or an increased transport capacity for reactive oxygen species, reactive nitrogen species as well as endogenous and exogenous ligands, wherein said human albumin solution is obtained in the course of a cold ethanol precipitation process of human blood plasma, wherein said method comprises the steps of
(a) providing a human albumin solution comprising mercaptalbumin and non-mercaptalbumin, wherein said non-mercaptalbumin comprises a disulfide moiety at position Cys-34;
(b) combining glutathione, at least once, with said human albumin solution at a molecular ratio of albumin to glutathione (albumin:glutathione) in the range of 1:1.5 to 1:10; and
(c) converting at least part of said non-mercaptalbumin into mercaptalbumin at a temperature of less than 15°C,
   wherein said mercaptalbumin content in said human albumin solution and/or said transport capacity for reactive oxygen species, reactive nitrogen species as well as endogenous and exogenous ligands of said human albumin solution is increased relative to the mercaptalbumin content of a human albumin solution prepared in a corresponding cold ethanol precipitation process of human blood plasma lacking steps (b) and (c).

In a second aspect, the present invention relates to a human albumin solution comprising an increased mercaptalbumin content when prepared by the method according to the first aspect, characterised in that it comprises a content of mercaptalbumin of at least 80%, in particular of at least 85%, relative to the total albumin content of said solution.

In a third aspect, the present invention relates to a human albumin solution of the second aspect for use in the treatment of a patient in need thereof.

Accordingly, this third aspect of the present invention also encompasses methods of treatment, wherein the method comprises administering human albumin solution of the second aspect to a patient in need thereof and/or use of a human albumin solution of the second aspect in the manufacture of a medicament for the treatment of a patient in need thereof.

Typically, the treatment comprises administering the human albumin solution having an increased mercaptalbumin content of the second aspect to a patient:
- suffering from hepatic failure; and/or
- suffering from chronic hepatitis, acute hepatitis, liver cirrhosis, fulminant hepatic failure or a hepatocellular carcinoma; and/or
- suffering from renal failure; and/or
- suffering from a blood volume deficiency and for restoration and maintenance of the circulating blood volume in said patient.

In a fourth aspect, the present invention relates to use of glutathione for increasing the mercaptalbumin content the transport capacity for reactive oxygen species, reactive nitrogen species as well as endogenous and exogenous ligands of a human albumin solution, wherein said human albumin solution is obtained in the course of a cold ethanol precipitation process of human blood plasma during which glutathione is added at least once, wherein said use comprises:
(a) providing a human albumin solution comprising mercaptalbumin and non-mercaptalbumin, wherein said non-mercaptalbumin comprises a disulfide moiety at position Cys-34;
(b) combining glutathione, at least once, with said human albumin solution at a molecular ratio of albumin to glutathione (albumin:glutathione) in the range of 1:1.5 to 1:10; and
(c) converting at least part of said non-mercaptalbumin into mercaptalbumin at a temperature of less than 15°C,
   wherein said mercaptalbumin content in said human albumin solution and/or said transport capacity for reactive oxygen species, reactive nitrogen species as well as endogenous and exogenous ligands of said human albumin solution is increased relative to the mercaptalbumin content of a human albumin solution prepared in a corresponding cold ethanol precipitation process of human blood plasma lacking steps (b) and (c).

In a fifth aspect, the present invention relates to a human albumin solution comprising an increased mercaptalbumin content when obtained through the use of glutathione according to the fourth aspect, characterised in that it comprises a content of mercaptalbumin of at least 80%, in particular of at least 85%, relative to the total albumin content of said solution.

In a sixth aspect, the present invention relates to a human albumin solution of the fifth aspect for use in the treatment of a patient in need thereof.

Accordingly, this sixth aspect of the present invention also encompasses methods of treatment, wherein the method comprises administering human albumin solution to a patient in need thereof and/or use of a human albumin solution in the manufacture of a medicament for the treatment of a patient in need thereof.

Typically, the treatment comprises administering the human albumin solution of the fifth aspect to a patient:
- suffering from hepatic failure; and/or
- suffering from chronic hepatitis, acute hepatitis, liver cirrhosis, fulminant hepatic failure or a hepatocellular carcinoma; and/or
- suffering from renal failure; and/or
   suffering from a blood volume deficiency and for restoration and maintenance of the circulating blood volume in said patient.

### DETAILED DESCRIPTION OF THE INVENTION

In order to provide a clear and consistent understanding of the specification and claims, and the scope to be given such terms, the following definitions are provided.

In the context of the present disclosure, the terms **"albumin", "human albumin"** or **"human serum albumin"** refer to unmodified human serum albumin (UniProtKB P02768; ALBU_HUMAN; https://www.uniprot.org/uniprotkb/P02768/entry) exclusively. The terms consequently do not encompass any modifications of albumin, such as fusion proteins (whether comprising full-length human serum albumin or fragments thereof) or any conjugates of human serum albumin with other molecules (e.g. PEG or bioactive molecules).

The term **"Cohn process"** refers to the well-known series of purification steps with the purpose of extracting albumin from blood plasma developed by Edwin J. Cohn. The process is based on the differential solubility of albumin and other plasma proteins based on pH, ethanol concentration, temperature, ionic strength, and protein concentration as published in 1946 (Cohn, E. J. et al. 1946 'Preparation and Properties of Serum and Plasma Proteins. IV. A System for the Separation into Fractions of the Protein and Lipoprotein Components of Biological Tissues and Fluids1a,b,c,d". Journal of the American Chemical Society. 68 (3): 459-475.

The term **"Kistler-Nitschmann process"** refers to the also well-known process for the large-scale production of human plasma fractions including a series of alcohol precipitations as published in 1962 (Kistler & Nitschmann 1962 "Large scale production of human plasma fractions. Eight years experience with the alcohol fractionation procedure of Nitschmann, Kistler and Lergier". Vox Sang. 1962 Jul-Aug;7:414-24).

Unless the context clearly requires otherwise, throughout the description and the claims, the words **"comprise", "comprising",** and the like are to be construed in an inclusive sense as opposed to an exclusive or exhaustive sense; that is to say, in the sense of "including, but not limited to".

Further, reference throughout this specification to **"one embodiment", "some embodiments"** or **"an embodiment"** means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives **"first", "second", "third",** etc., to describe a common object, merely indicate that different instances of like objects are being referred to, and are not intended to imply that the objects so described must be in a given sequence, either temporally, spatially, in ranking, or in any other manner.

As used herein, the term **"exemplary"** is used in the sense of providing examples, as opposed to indicating quality. That is, an **"exemplary embodiment"** is an embodiment provided as an example, as opposed to necessarily being an embodiment of exemplary quality.

As already mentioned above, in plasma, mercaptalbumin conveys several benefits, including:
- antioxidant protection: mercaptalbumin can act as an antioxidant, helping to protect cells and tissues from damage caused by free radicals and reactive oxygen species;
- detoxification: mercaptalbumin can bind to and help remove toxic substances from the body, such as heavy metals and drugs; and
- immune function: mercaptalbumin can help to stimulate the immune system and protect against infections.

Consequently, a high percentage of non-mercaptalbumin can lead to a decreased ability to protect against oxidative damage, detoxify harmful substances, and/or support immune function. Additionally, a low level of mercaptalbumin may be indicative of certain health conditions or diseases, such as liver disease or malnutrition.

However, as also already mentioned, commercial human albumin products, produced on an industrial scale, are known to comprise lower amounts of beneficial mercaptalbumin and higher amounts of both non-desirable non-mercaptalbumins than plasma. Further, commercially available albumins are formulated with substantial amounts (i.e. an excess) of fatty compounds such as tryptophan and/or caprylic acid. As a result, the ratio between albumin and such fatty compounds in commercially available albumin solutions is routinely between 1:5 aand 1:8. For example, the commercially available 5% Octalbin albumin solution comprises 5% albumin (i.e. 50 g/l; 0.75 mmol) as well as tryptophan and caprylic acid each at concentrations of up to 4.2 mmol. The 25% Octalbin albumin solution comprises 25% albumin (i.e. 250 g/l; 3.8 mmol) as well as tryptophan and caprylic acid each at concentrations of up to 21 mmol. As such, and without being bound by theory, an excess of fatty compounds likely leads to the majority of the albumin's Fatty Acid binding sites FA1 to FA7 to be occupied thereby further limiting the albumin's transport capacity for reactive oxygen species, reactive nitrogen species as well as endogenous and exogenous ligands.

As the conversion from mercaptalbumin to non-mercaptalbumin-1 is the result of the oxidation of the thiol group at Cys-34 of albumin, it is not entirely surprising that during the industrial scale purification process of albumin from batches of pooled donor plasma, typically based on the Cohn process or the Kistler-Nitschmann process, and comprising serial precipitation and reconstitution steps, a greater degree of Cys-34 oxidation is seen in the albumin end product of the purification process compared to fresh plasma.

However, as mercaptalbumin has the above-mentioned beneficial properties, methods and procedures to increase the mercaptalbumin content of industrially purified albumin products are needed.

The methods of albumin preparation and uses according to the present invention achieve an increased mercaptalbumin content relative to the mercaptalbumin content of a human albumin solution prepared using the known methods. Furthermore, however, the methods of albumin preparation and uses according to the present invention can even achieve an increased mercaptalbumin content relative to the mercaptalbumin content of plasma, i.e. a mercaptalbumin content of greater than 80%.

### Methods of the invention and related albumin products

In the first aspect, the present invention relates to a method for the preparation of a human albumin solution having an increased mercaptalbumin content and/or an increased transport capacity for reactive oxygen species, reactive nitrogen species as well as endogenous and exogenous ligands, wherein said human albumin solution is obtained in the course of a cold ethanol precipitation process of human blood plasma, wherein said method comprises the steps of
(a) providing a human albumin solution comprising mercaptalbumin and non-mercaptalbumin, wherein said non-mercaptalbumin comprises a disulfide moiety at position Cys-34;
(b) combining glutathione, at least once, with said human albumin solution at a molecular ratio of albumin to glutathione (albumin:glutathione) in the range of 1:1.5 to 1:10; and
(c) converting at least part said non-mercaptalbumin into mercaptalbumin at a temperature of less than 15°C,
   wherein said mercaptalbumin content in said human albumin solution and/or said transport capacity for reactive oxygen species, reactive nitrogen species as well as endogenous and exogenous ligands of said human albumin solution is increased relative to the mercaptalbumin content of a human albumin solution prepared in a corresponding cold ethanol precipitation process of human blood plasma lacking steps (b) and (c).

The endogenous and exogenous ligands albumin are typically selected from fatty acids (FAs), nucleic acids, hormones, metals, toxins and drugs, wherein such drugs specifically include ibuprofen, diazepam, warfarin, lidocaine and indomethacine.

As indicated, the conversion step (c) of the above method is advantageously performed at relatively low temperatures of less than 15°C. While one would expect that the conversion is less efficient at lower temperatures, it has been found that the lower temperatures strike the desired balance between a slower desirable conversion of non-mercaptalbumin to mercaptalbumin and slower undesirable oxidation of Cys-34 such that the mercaptalbumin content can be increased as described. The temperature at which step (c) is performed is, in some instances, less than 14°C, less than 13°C, less than 12°C, less than 11°C, less than 10°C, less than 9°C, less than 8°C, less than 7°C, less than 6°C, less than 5°C, less than 4°C, less than 3°C, less than 2°C, less than 1°C, less than 0°C, less than -1°C, less than -2°C, less than -3°C, less than -4°C, less than -5°C, less than -6°C, less than -7°C, less than -8°C, less than -9°C, less than -10°C.

The temperature at which step (c) is performed, in some instances, ranges between -25°C and -22°C, between -22°C and -19°C, between -19°C and -16°C, between - 16°C and -13°C, between -13°C and -10°C, between -10°C and -7°C, between -7°C and -4°C, between -4°C and -1°C, between -1°C and 2°C, between 2°C and 5°C, between 5°C and 8°C, between 8°C and 11°C, between 11 °C and 14°C or between - 25°C to -23°C, between -23°C to -21°C, between -21°C to -19°C , between -19°C to -17°, between -17°C to -15°C, between -15°C to -13°C, between -13°C to -11°C, between -11°C to -9°C, between -9°C to -7°C, between -7°C to -5°C, between -5°C to -3°C, between -3°C to -1°C, between -1°C to 1°C, between 1°C to 3°C, between 3°C to 5°, between 5°C to 7°, between 7°C to 9°, between 9°C to 11°, between 11°C to 13°C or between 13°C to 15°C. Preferably, between -10°C to +10°C. The temperature at which step (c) is performed is, in some instances, 15°C, 14°C, 13°C, 12°C, 11°C, 10°C, 9°C, 8°C, 7°C, 6°C, 5°C, 4°C, 3°C, 2°C, 1°C, 0°C, -1°C, -2°C, - 3°C, -4°C, -5°C, -6°C, -7°C, -8°C, -9°C, -10°C, -11°C, -12°C, -13°C, -14°C, -15°C, - 16°C, -17°C, -18°C, -19°C, -20°C, -21°C, -22°C, -23°C, -24°C, or -25°C.

Preferably, the molecular ratio of albumin to glutathione (albumin:glutathione) is in the range of 1:2 to 1:5, such as 1:2, 1:3, 1:4 or 1:5.

In some embodiments of the method of the first aspect, step (b), optionally also step (c), precedes at least one further time-consuming step selected from the group consisting of: a liquid-solid separation step, preferably a filtration, sedimentation, precipitation or centrifugation step; a reconstitution step; a resuspension step; a concentration step, preferably an ultra- or diafiltration step; and a recovery from storage step.

In the context of the present disclosure, it is noteworthy that, for example the simple process of thawing a 2000 liter batch of frozen pooled donor plasma typically requires at least 3 hours of time, sometimes at least 5 hours of time.

Performing step (b), optionally also step (c), prior to either one (or several or all) of the time-consuming steps, which typically require at least 3 hours, sometimes at least 5 hours of time, in the conventional plasma fractionation processes (i.e. the Cohn process or the Kistler Nitschmann process and variations thereof) counters the unwanted oxidation of desirable mercaptalbumin to undesirable non-mercaptalbumin during the course of each of the respective step (or during the course of each of these respective steps) of the respective process. This has the advantage that the final albumin product has an increased mercaptalbumin content compared to an albumin product, simply prepared by the conventional processes.

Preferably, the method further comprises providing said glutathione to be combined with the human albumin comprising solution in step (b) in a vessel prior to the addition of human blood plasma or human whole blood to the vessel and wherein, optionally, said human blood plasma or human whole blood is stored in the vessel. As such, the glutathione may already be combined with the starting material of the Cohn or Kistler Nitschmann processes at the time of donation or shortly thereafter, when the whole blood or plasma (i.e. the human albumin solution) is filled into a plasma bag or bottle. In some or all of the above embodiments of the method of the first aspect, step (b) may precede a step of harvesting cryo-poor plasma. If so, the method further comprises providing the glutathione to be combined with the human albumin comprising solution in step (b) in a vessel prior to:
- pooling frozen human plasma donations in the vessel;
- thawing the frozen human plasma donations at a temperature of 0°C to 5°C;
- separating still solid cryoprecipitate from the liquid cryo-poor plasma; and
- optionally, further processing the liquid cryo-poor plasma by capturing coagulation factors to generate a clotting factor-poor plasma.

The step of thawing of several cryo-poor plasma batches to be pooled regularly takes between 3 and 10 hours of time, such as between 4 and 10 hour, 5 and 10 hours, 6 and 10 hours, 7 and 10 hours, 8 and 10 hours or 9 and 10 hours.

Once the solid cryoprecipitate is separated from the liquid cryo-poor plasma, the liquid cryo-poor plasma can be further processed on synthetic resins such as Sephadex or Heparin-coupled resins to capture coagulation Factor IX, the PPSB or FXI. PPSB stands for Prothrombin Complex Concentrate (PCC) or Prothrombin Complex Concentrate with Factor VIII Inhibitor Bypassing Activity and typically comprises coagulation Factors II, VII, IX, and X.

In some or all of the above embodiments of the method of the first aspect, step (b) precedes a step of forming Fraction I+II+III of the Cohn process or of forming Precipitate A of the Kistler-Nitschmann (KN) process and wherein the method further comprises adding the glutathione to be combined with the human albumin solution to cryo-poor plasma or to coagulation factor-poor plasma prior to the addition of ethanol. Forming Fraction I+II+III of the Cohn process or forming Precipitate A of the Kistler-Nitschmann (KN) process typically comprises the use of filter aids as well as a reduction in temperature to less than -1°C, such as to between -1°C and -6°C. The entire step comprises several sub-steps making it time consuming, often requiring up to 32 hours of time.

In some or all of the above embodiments of the method of the first aspect, step (b) precedes a step of forming Fraction IV of the Cohn process or of forming Precipitate A of the KN process, wherein the method further comprises adding the glutathione to be combined with the human albumin solution to the Fraction I+II+III supernatant or to the Precipitate A supernatant prior to the addition of ethanol. Forming Fraction IV of the Cohn process or forming Precipitate A of the KN process typically comprises the use of filter aids as well as a reduction in temperature to less than -4°C, such as to between -4°C and -10°C. The entire step comprises several sub-steps making it time consuming, often requiring up to 11 hours of time such as between 8 and 11 hours of time (not including potential precipitation steps to capture Factor IV).

In some or all of the above embodiments of the method of the first aspect, step (b) precedes a step of forming Fraction V of the Cohn process or of forming Precipitate A of the KN process, wherein the method further comprises adding the glutathione to be combined with the human albumin solution to the Fraction IV supernatant prior to addition of ethanol. Forming Fraction V of the Cohn process or forming Precipitate A of the KN process typically does not comprise the use of filter aids but also a reduction in temperature to less than -8°C, such as to between -8°C and -10°C. The entire step comprises several sub-steps making it time consuming, often requiring up to 62 hours of time such as between 18 and 62 hours of time, including certain holding times.

In some or all of the above embodiments of the method of the first aspect, step (b) precedes a step of resuspending Fraction V of the Cohn process or of resuspending Precipitate C of the KN process, wherein the method further comprises adding the glutathione to be combined with the human albumin solution to Fraction V prior to resuspension in ethanol or in water for injection (WFI)/ethanol. Resuspending Fraction V of the Cohn process or of resuspending Precipitate C of the KN process typically comprises an increase in temperature in temperature to less than +1°C, such as to between +1°C and -4°C. The entire step comprises several sub-steps making it time consuming, often requiring up to 22 hours of time such as between 7.5 and 22 hours of time.

Typically, the method further comprises a final adjustment of the protein content of the human albumin solution during which one or more stabilizers selected from the group consisting of: amino acids; sugars; and sugar alcohols, are optionally added.

Preferably, the one or more stabilizers are selected from the group consisting of: glycine; glutamate; arginine; lysine; maltose; and sorbitol.

Preferably, the method further comprises performing pathogen inactivation or pathogen removal, in particular virus inactivation or virus removal, on the human albumin solution, preferably by performing nanofiltration, solvent/detergent treatment (S/D treatment), low pH treatment or pasteurization of the human albumin solution, or a combination thereof.

Generally, pasteurization of the human albumin solution is performed as a one-step or as a two-step process, optionally under an inert atmosphere. The one-step pasteurization process is performed in the final container comprising stabilizers N-acetyl-tryptophan and caprylic acid, while the two-step pasteurization process comprises an additional pasteurization with the same stabilizers just prior to filling albumin into the final containers.

If an S/D treatment is employed, preferably, an environmentally-friendly detergent, i.e. a detergent other than Triton X-100, is used. For example, polyethylene glycol ether, sec-alcohol ethoxylate, sec-alcoxy polyethylene glycol (Tergitol 15-S-9), Nereid or a polysorbate, such as polysorbate 20 or polysorbate 80 (TWEEN 20 or TWEEN 80). However, in embodiments of the present invention where the removal of any S/D reagents is performed without an oil extraction, i.e. in some embodiments of the present invention an S/D treatment does not include an oil extraction step. Instead, undesirable S/D reagents are removed using other purification means available to the skilled person such as, for example, by (a) chromatographic separation such as by using chromatography gels or resins, (b) fractionation methods such as precipitation methods or (c) filtration such as by diafiltration or by using adsorbents such as activated charcoal.

If an S/D treatment is employed, the S/D treatment may replace pasteurization of the human albumin solution as virus inactivation step. Accordingly, the S/D treatment performed on the human albumin solution for virus inactivation, which is suitable to replace pasteurization entirely, is performed without the addition of an indole stabilizer (such as N-acetyl-tryptophan) or a fatty acid stabilizer (such as caprylate). Instead, alternative stabilizers may be used, for example, stabilizers selected from the group of: sugars, amino acids, and sugar alcohols. Preferably, the one or more stabilizers are selected from the group consisting of: glycine; glutamate; arginine; lysine; maltose; and sorbitol.

In some embodiments, the virus inactivation is performed by employing a low pH treatment. Many viruses are irreversibly denatured and effectively destroyed at pH 5.0-5.5. However, several enveloped viruses are only effectively inactivated at pH range 3.5-4. Accordingly, the low pH treatment in the context of the present invention is treatment at a pH of 5.5 or less, preferably at a pH between 3.0 and 5.5, such as at a pH of about 3.0, 3.5, 4.0, 4.5, 5.0 or 5.5.

In some embodiments, the low pH treatment at pH range 3.0-5.5 serves as a degreasing step for albumin. A simultaneous virus inactivation and degreasing of albumin is thus possible without the necessity to remove virus inactivating compounds. Simple pH adjustment after low pH treatment to albumin compatible values is sufficient.

Preferably, the method for the preparation of the human albumin solution comprising an increased mercaptalbumin content and/or an increased transport capacity for reactive oxygen species, reactive nitrogen species as well as endogenous and exogenous ligands comprises filling the solution into a final container, which is subsequently eventually flushed with an inert gas.

### Uses of the invention

In a fourth aspect, the present invention relates to use of glutathione for increasing the mercaptalbumin content and/or an increased transport capacity for reactive oxygen species, reactive nitrogen species as well as endogenous and exogenous ligands of a human albumin solution, wherein said human albumin solution is obtained in the course of a cold ethanol precipitation process of human blood plasma during which glutathione is added at least once, wherein said use comprises:
(a) providing a human albumin solution comprising mercaptalbumin and non-mercaptalbumin, wherein said non-mercaptalbumin comprises a disulfide moiety at position Cys-34;
(b) combining glutathione, at least once, with said human albumin solution at a molecular ratio of albumin to glutathione (albumin:glutathione) in the range of 1:1.5 to 1:10, wherein; and
(c) converting at least part of said non-mercaptalbumin into mercaptalbumin at a temperature of less than 15°C,
wherein said mercaptalbumin content in said human albumin solution and/or said transport capacity for reactive oxygen species, reactive nitrogen species as well as endogenous and exogenous ligands of said human albumin solution is increased relative to the mercaptalbumin content of a human albumin solution prepared in a corresponding cold ethanol precipitation process of human blood plasma lacking steps (b) and (c).

As indicated, the conversion of (c) is advantageously relatively low and less than 15°C. While one would expect that the conversion is less efficient at lower temperatures, it has been found that the lower temperatures strike the desired balance between a slower desirable conversion of non-mercaptalbumin to mercaptalbumin and slower undesirable oxidation of Cys-34 such that the mercaptalbumin content can be increased as described. The temperature at of (c) is, in some instances, less than 14°C, less than 13°C, less than 12°C, less than 11°C, less than 10°C, less than 9°C, less than 8°C, less than 7°C, less than 6°C, less than 5°C, less than 4°C, less than 3°C, less than 2°C, less than 1°C, less than 0°C, less than -1°C, less than -2°C, less than -3°C, less than -4°C, less than -5°C, less than - 6°C, less than -7°C, less than -8°C, less than -9°C, less than -10°C.

The temperature of (c), in some instances, ranges between -25°C and -22°C, between -22°C and -19°C, between -19°C and -16°C, between -16°C and -13°C, between - 13°C and -10°C, between -10°C and -7°C, between -7°C and -4°C, between -4°C and -1°C, between -1°C and 2°C, between 2°C and 5°C, between 5°C and 8°C, between 8°C and 11°C, between 11°C and 14°C or between -25°C to -23°C, between -23°C to -21°C, between -21°C to -19°C , between -19°C to -17°, between -17°C to -15°C, between -15°C to -13°C, between -13°C to -11°C, between -11°C to -9°C, between - 9°C to -7°C, between -7°C to -5°C, between -5°C to -3°C, between -3°C to -1°C, between -1°C to 1°C , between 1°C to 3°C, between 3°C to 5°, between 5°C to 7°, between 7°C to 9°, between 9°C to 11°, between 11°C to 13°C or between 13°C to 15°C. Preferably, between -10°C to +10°C. The temperature of (c) is, in some instances, 15°C, 14°C, 13°C, 12°C, 11°C, 10°C, 9°C, 8°C, 7°C, 6°C, 5°C, 4°C, 3°C, 2°C, 1°C, 0°C, -1°C, -2°C, -3°C, -4°C, -5°C, -6°C, -7°C, -8°C, -9°C, -10°C, -11°C, - 12°C, -13°C, -14°C, -15°C, -16°C, -17°C, -18°C, -19°C, -20°C, -21°C, -22°C, -23°C, - 24°C, or -25°C.

Preferably, the molecular ratio of albumin to glutathione (albumin:glutathione) is in the range of 1:2 to 1:5, such as 1:2, 1:3, 1:4 or 1:5.

In some embodiments, (b), optionally also (c), precedes at least one further time-consuming step selected from the group consisting of: a liquid-solid separation step, preferably a filtration, sedimentation, precipitation or centrifugation step; a reconstitution step; a resuspension step; a concentration step, preferably an ultra- or diafiltration step; and a recovery from storage step.

Performing (b), optionally also (c), prior to either one (or several or all) of the time-consuming steps, which typically require at least 3 hours, sometimes at least 5 hours of time, in the conventional plasma fractionation processes (i.e. the Cohn process or the Kistler-Nitschmann process and variations thereof) counters the unwanted oxidation of desirable mercaptalbumin to undesirable non-mercaptalbumin during the course of each of the respective step (or during the course of each of these respective steps) of the respective process. This has the advantage that an increased mercaptalbumin content can be achieved through the use of glutathione in a human albumin solution compared to a human albumin solution, not treated with glutathione as described but which has simply undergone conventional processing.

Preferably, the glutathione to be combined with the human albumin comprising solution in (b) may be used in a vessel prior to the addition of human blood plasma or human whole blood to the vessel such that, optionally, said human blood plasma or human whole blood can be stored in the vessel.

In some or all of the above embodiments of the fourth aspect, (b) may precede a step of harvesting cryo-poor plasma. If so, the method further comprises providing the glutathione to be combined with the human albumin comprising solution in (b) in a vessel prior to:
- pooling frozen human plasma donations in the vessel;
- thawing the frozen human plasma donations at a temperature of 0°C to 5°C;
- separating still solid cryoprecipitate from the liquid cryo-poor plasma; and
- optionally, further processing the liquid cryo-poor plasma by capturing coagulation factors to generate a clotting factor-poor plasma.

In some or all of the above embodiments of the fourth aspect, (b) precedes a step of forming Fraction I+II+III of the Cohn process or of forming Precipitate A of the Kistler-Nitschmann (KN) process and wherein the method further comprises adding the glutathione to be combined with the human albumin solution to cryo-poor plasma or to coagulation factor-poor plasma prior to the addition of ethanol.

In some or all of the above embodiments of the fourth aspect, (b) precedes a step of forming Fraction IV of the Cohn process or of forming Precipitate A of the KN process, wherein the method further comprises adding the glutathione to be combined with the human albumin solution to the Fraction I+II+III supernatant or to the Precipitate A supernatant prior to the addition of ethanol.

In some or all of the above embodiments of the fourth aspect, (b) precedes a step of forming Fraction V of the Cohn process or of forming Precipitate A of the KN process, wherein the method further comprises adding the glutathione to be combined with the human albumin solution to the Fraction IV supernatant prior to addition of ethanol.

In some or all of the above embodiments of the fourth aspect, (b) precedes a step of resuspending Fraction V of the Cohn process or of resuspending Precipitate C of the KN process, wherein the method further comprises adding the glutathione to be combined with the human albumin solution to Fraction V prior to resuspension in ethanol or in water for injection (WFI)/ethanol.

Typically, the use further comprises a final adjustment of the protein content of the human albumin solution during which one or more stabilizers selected from the group consisting of: amino acids; sugars; and sugar alcohols, are optionally added. Preferably, the one or more stabilizers are selected from the group consisting of: glycine; glutamate; arginine; lysine; maltose; and sorbitol.

Preferably, the use further comprises performing pathogen inactivation or pathogen removal, in particular virus inactivation or virus removal, on the human albumin solution, preferably by performing nanofiltration, solvent/detergent treatment (S/D treatment), low pH treatment or pasteurization of the human albumin solution, or a combination thereof.

Generally, pasteurization of the human albumin solution is performed as a one-step or as a two-step process, optionally under an inert atmosphere.

If an S/D treatment is employed, preferably, an environmentally-friendly detergent, i.e. a detergent other than Triton X-100, is used. For example, polyethylene glycol ether, sec-alcohol ethoxylate, sec-alcoxy polyethylene glycol (Tergitol 15-S-9), Nereid or a polysorbate, such as polysorbate 20 or polysorbate 80 (TWEEN 20 or TWEEN 80). However, in embodiments of the present invention where the removal of any S/D reagents is performed without an oil extraction, i.e. in some embodiments of the present invention an S/D treatment does not include an oil extraction step. Instead, undesirable S/D reagents are removed using other purification means available to the skilled person such as, for example, by (a) chromatographic separation such as by using chromatography gels or resins, (b) fractionation methods such as precipitation methods or (c) filtration such as by diafiltration or by using adsorbents such as activated charcoal.

If an S/D treatment is employed, the S/D treatment may replace pasteurization of the human albumin solution as virus inactivation step. Accordingly, the S/D treatment performed on the human albumin solution for virus inactivation, which is suitable to replace pasteurization entirely, is performed without the addition of an indole stabilizer (such as N-acetyl-tryptophan) or a fatty acid stabilizer (such as caprylate). Instead, alternative stabilizers may be used, for example, stabilizers selected from the group of: sugars, amino acids, and sugar alcohols. Preferably, the one or more stabilizers are selected from the group consisting of: glycine; glutamate; arginine; lysine; maltose; and sorbitol.

In some embodiments, the virus inactivation and degreasing is performed by employing a low pH treatment. Many viruses are irreversibly denatured and effectively destroyed at pH 5.0-5.5. However, several enveloped viruses are only effectively inactivated at pH range 3.5-4. Accordingly, the low pH treatment in the context of the present invention is treatment at a pH of 5.5 or less, preferably at a pH between 3.0 and 5.5, such as at a pH of about 3.0, 3.5, 4.0, 4.5, 5.0 or 5.5.

Preferably, the use of glutathione for increasing the mercaptalbumin content and/or for increasing the transport capacity for reactive oxygen species, reactive nitrogen species as well as endogenous and exogenous ligands of the human albumin solution comprising an increased mercaptalbumin content and/or an increased transport capacity for reactive oxygen species, reactive nitrogen species as well as endogenous and exogenous ligands comprises filling the solution into a final container, which is subsequently flushed with an inert gas.

In a fifth aspect, the present invention relates to a human albumin solution comprising an increased mercaptalbumin content when obtained through the use according to the fourth aspect as described above, characterised in that the human albumin solution comprises a content of mercaptalbumin of at least 80%, in particular of at least 85%, relative to the total albumin content of the solution.

The human albumin solutions of the present invention can be used in the treatment of disease. Specifically, it is for use in the treatment of a patient in need thereof. In particular for use of the treatment of a patient:
- suffering from hepatic failure; and/or
- suffering from chronic hepatitis, acute hepatitis, liver cirrhosis, fulminant hepatic failure or a hepatocellular carcinoma; and/or
- suffering from renal failure; and/or
- suffering from a blood volume deficiency and for restoration and maintenance of the circulating blood volume in said patient.

### Albumin products of the invention

In the second and third aspect as well as in the fifth and sixth aspects, the present invention relates to human albumin solutions comprising an increased mercaptalbumin content and/or an increased transport capacity for reactive oxygen species, reactive nitrogen species as well as endogenous and exogenous ligands of said human albumin solution when prepared by the method according to the first aspect or the use according to the fourth aspect, respectively.

The respective human albumin solutions can be used as a pharmaceutical.

Specifically, the respective human albumin solutions are for use in the treatment of a patient in need thereof. In particular for use of the treatment of a patient:
- suffering from hepatic failure; and/or
- suffering from chronic hepatitis, acute hepatitis, liver cirrhosis, fulminant hepatic failure or a hepatocellular carcinoma; and/or
- suffering from renal failure; and/or
   suffering from a blood volume deficiency and for restoration and maintenance of the circulating blood volume in said patient.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

## Claims

1. A method for the preparation of a human albumin solution having an increased mercaptalbumin content and/or an increased transport capacity for reactive oxygen species, reactive nitrogen species as well as endogenous and exogenous ligands, wherein said human albumin solution is obtained in the course of a cold ethanol precipitation process of human blood plasma, wherein said method comprises the steps of
(a) providing a human albumin solution comprising mercaptalbumin and non-mercaptalbumin, wherein said non-mercaptalbumin comprises a disulfide moiety at position Cys-34;
(b) combining glutathione, at least once, with said human albumin solution at a molecular ratio of albumin to glutathione (albumin:glutathione) in the range of 1:1.5 to 1:10; and
(c) converting at least part of said non-mercaptalbumin into mercaptalbumin at a temperature of less than 15°C,
wherein said mercaptalbumin content in said human albumin solution and/or said transport capacity for reactive oxygen species, reactive nitrogen species as well as endogenous and exogenous ligands of said human albumin solution is increased relative to the mercaptalbumin content of a human albumin solution prepared in a corresponding cold ethanol precipitation process of human blood plasma lacking steps (b) and (c).

2. The method according to claim 1, wherein the molecular ratio of albumin to glutathione (albumin:glutathione) is in the range of 1:2 to 1:5.

3. The method according to claim 1 or claim 2, wherein step (b) precedes at least one further time-consuming step selected from the group consisting of: a liquid-solid separation step, preferably a filtration, sedimentation, precipitation or centrifugation step; a reconstitution step; a resuspension step; a concentration step, preferably an ultra- or diafiltration step; and a recovery from storage step, and wherein, optionally, said time-consuming step requires at least 3 hours, preferably at least 5 hours, of time.

4. The method according to any one of claims 1 to 3, wherein said method further comprises providing said glutathione to be combined with said human albumin solution in step (b) in a vessel prior to the addition of human blood plasma or human whole blood to said vessel and wherein, optionally, said human blood plasma or human whole blood is stored in said vessel.

5. The method according to any one of claims 1 to 3, wherein step (b) precedes a step of harvesting cryo-poor plasma and wherein said method further comprises providing said glutathione to be combined with said human albumin comprising solution in step (b) in a vessel prior to:
- pooling frozen human plasma donations in said vessel;
- thawing said frozen human plasma donations at a temperature of 0°C to 5°C;
- separating still solid cryoprecipitate from said liquid cryo-poor plasma; and
- optionally, further processing said liquid cryo-poor plasma by capturing coagulation factors to generate a clotting factor-poor plasma.

6. The method according to any one of claims 1 to 5, wherein:
step (b) precedes a step of forming Fraction I+II+III of the Cohn process or of forming Precipitate A of the Kistler-Nitschmann (KN) process and wherein said method further comprises adding said glutathione to be combined with said human albumin solution to cryo-poor plasma or to coagulation factor-poor plasma prior to the addition of ethanol; and/or
wherein step (b) precedes a step of forming Fraction IV of the Cohn process or of forming Precipitate A of the KN process, wherein said method further comprises adding said glutathione to be combined with said human albumin solution to the Fraction I+II+III supernatant or to the Precipitate A supernatant prior to the addition of ethanol; and/or
step (b) precedes a step of forming Fraction V of the Cohn process or of forming Precipitate A of the KN process, wherein said method further comprises adding said glutathione to be combined with said human albumin solution to the Fraction IV supernatant prior to addition of ethanol; and/or
step (b) precedes a step of resuspending Fraction V of the Cohn process or of resuspending Precipitate C of the KN process, wherein said method further comprises adding said glutathione to be combined with said human albumin solution to Fraction V prior to resuspension in ethanol or in WFI/ethanol.

7. The method according to any one of claims 1 to 6, wherein said method further comprises a final adjustment of the protein content of said human albumin solution during which one or more stabilizers selected from the group consisting of: amino acids; sugars; and sugar alcohols, are optionally added.

8. The method according to claim 7, wherein the one or more stabilizers are selected from the group consisting of: glycine; glutamate; arginine; lysine; maltose; and sorbitol.

9. The method according to any one of claims 1 to 8, wherein said method further comprises performing pathogen inactivation or pathogen removal, in particular virus inactivation or virus removal, on said human albumin solution, preferably by performing nanofiltration, solvent/detergent treatment (S/D treatment), low pH treatment or pasteurization of said human albumin solution, or a combination thereof, and,
optionally, pasteurization of said human albumin solution is performed as a one-step or as a two-step process, optionally under an inert atmosphere.

10. The method according to any one of claims 1 to 9, comprises filling said solution into a final container, which is subsequently flushed with an inert gas.

11. The method according to any one of claims 1 to 10, wherein
(a) said reactive oxygen species are selected from the group consisting of: H₂O₂, O₂⁻ and HOCl;
(b) said reactive nitrogen species are selected from the group consisting of: ONOO- and ONOOCO₂⁻;
(c) endogenous and exogenous ligands are selected from the group consisting of: fatty acids (FAs), nucleic acids, hormones, metals, toxins and drugs.

12. A human albumin solution comprising an increased mercaptalbumin content when prepared by the method according to any one of claims 1 to 11, **characterised in that** it comprises a content of mercaptalbumin of at least 80%, in particular of at least 85%, relative to the total albumin content of said solution.

13. The human albumin solution comprising an increased mercaptalbumin content of claim 12 for use in the treatment of a patient in need thereof.

14. The human albumin solution comprising an increased mercaptalbumin content of claim 12 for use in the treatment of a patient suffering from hepatic failure.

15. The human albumin solution comprising an increased mercaptalbumin content for use according to claim 14, wherein said use comprises administering the human albumin solution to a patient suffering from chronic hepatitis, acute hepatitis, liver cirrhosis, fulminant hepatic failure or a hepatocellular carcinoma.

16. The human albumin solution comprising an increased mercaptalbumin content of claim 12 for use in the treatment of a patient suffering a renal failure.

17. The human albumin solution comprising an increased mercaptalbumin content of claim 12 for use in the restoration and maintenance of circulating blood volume in a patient, where blood volume deficiency has been demonstrated in said patient.

18. Use of glutathione for increasing the mercaptalbumin content of a human albumin solution, wherein said human albumin solution is obtained in the course of a cold ethanol precipitation process of human blood plasma during which glutathione is added at least once, wherein said use comprises:
(a) providing a human albumin solution comprising mercaptalbumin and non-mercaptalbumin, wherein said non-mercaptalbumin comprises a disulfide moiety at position Cys-34;
(b) combining glutathione, at least once, with said human albumin solution at a molecular ratio of albumin to glutathione (albumin:glutathione) in the range of 1:1.5 to 1:10; and
(c) converting said non-mercaptalbumin into mercaptalbumin at a temperature of less than 15°C,
wherein said mercaptalbumin content in said human albumin solution is increased relative to the mercaptalbumin content of a human albumin solution prepared in a corresponding cold ethanol precipitation process of human blood plasma lacking steps (b) and (c).

19. A human albumin solution comprising an increased mercaptalbumin content when obtained through the use of glutathione according to claim 17, being characterised that it comprises a content of mercaptalbumin of at least 80%, in particular of at least 85%, relative to the total albumin content of said solution.
